(19) Europäisches Patentamt
European Patent Office
Office européen des brevets

(11) Publication number: **0 001 401 A1**

(12) # EUROPEAN PATENT APPLICATION

(21) Application number: **78100861.0**

(22) Date of filing: **09.09.78**

(51) Int. Cl.$^2$: **C 07 D 495/04,** A 61 K 31/55 // (C07D495/04, 333/00, 223/00)

(30) Priority: **29.09.77 CH 11912/77**
**29.09.77 CH 11917/77**

(43) Date of publication of application: **18.04.79 Bulletin 79/8**

(84) Designated Contracting States: **BE CH DE FR GB LU NL SE**

(71) Applicant: **SANDOZ LTD., Lichtstrasse 35, CH-4002 Basel (CH)**

(72) Inventor: **Hunziker, Fritz, Dr., Wabernstrasse 59, CH-3000 Berne (CH)**

(54) **Thienobenzazepine derivatives, processes for their production and pharmaceutical compositions containing them.**

(57) Thienabenzosepines of formula I,

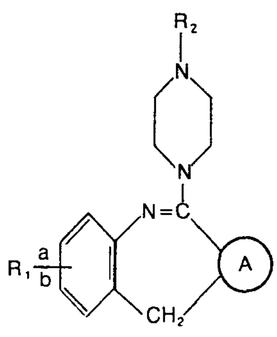

I

wherein

$R_1$ is hydrogen, halogen, trifluoromethyl or alkyl, alkoxy, alkylthio, alkylsulfinyl or alkylsulfonyl, each of 1 to 4 carbon atoms,

$R_2$ is hydrogen, alkyl of 1 to 4 carbon atoms, hydroxyalkyl with a maximum of 4 carbon atoms, which may be acylated by an alkanoyl group of 2 to 18 carbon atoms of alkoxyalkyl with a maximum of 6 carbon atoms, and

A is a radical of formula AI or AII

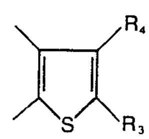

AI

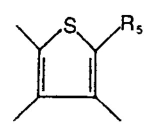

AII

wherein

$R_3$ is hydrogen, halogen, trifluoromethylthio, trifluoromethylsulfinyl, trifluoromethylsulfonyl or alkyl, alkoxy, alkylthio, alkylsulfinyl or alkylsulfonyl, each of 1 to 4 carbon atoms,

$R_4$ is hydrogen or alkyl of 1 to 4 carbon atoms, with the proviso that one of $R_3$ and $R_4$ is hydrogen and the other of $R_3$ and $R_4$ is other than hydrogen, and

$R_5$ is halogen or alkyl of 1 to 4 carbon atoms,

and their preparation.

(I) are useful for treating psychotic disturbances, depressions or relaxing muscles.

EP 0 001 401 A1

0001401

Case 500-5460

# THIENOBENZAZEPINE DERIVATIVES, PROCESSES FOR THEIR PRODUCTION AND PHARMACEUTICAL COMPOSITIONS CONTAINING THEM

The present invention relates to thienobenzazepines, processes for their production and pharmaceutical compositions containing them.

The present invention provides compounds of formula I,

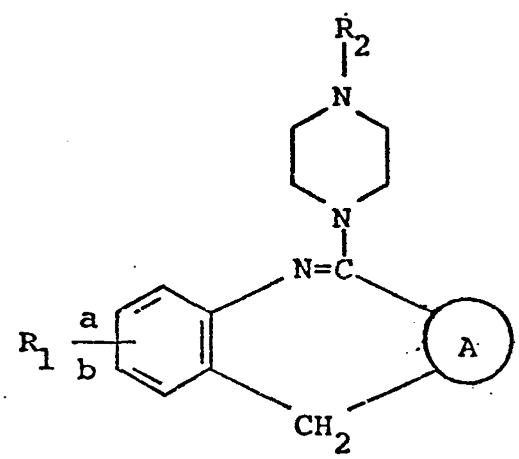

I

wherein $R_1$ is hydrogen, halogen, trifluoromethyl or alkyl, alkoxy, alkylthio, alkylsulfinyl or alkylsulfonyl, each of 1 to 4 carbon atoms,

$R_2$ is hydrogen, alkyl of 1 to 4 carbon atoms, hydroxyalkyl with a maximum of 4 carbon atoms, which may be acylated by an alkanoyl group of 2 to 18 carbon atoms or alkoxyalkyl with a maximum of 6 carbon atoms, and

A is a radical of formula AI or AII

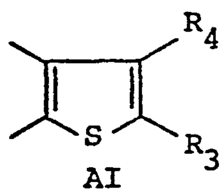

AI

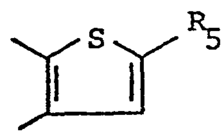

AII

wherein $R_3$ is hydrogen, halogen, trifluoromethylthio, trifluoromethylsulfinyl, trifluoromethylsulfonyl or alkyl, alkoxy, alkylthio, alkylsulfinyl or alkylsulfonyl, each of 1 to 4 carbon atoms,

$R_4$ is hydrogen or alkyl of 1 to 4 carbon atoms, with the proviso that one of $R_3$ and $R_4$ is hydrogen and the other of $R_3$ and $R_4$ is other than hydrogen, and

$R_5$ is halogen or alkyl of 1 to 4 carbon atoms.

Halogen means fluorine, chlorine, bromine or iodine, preferably fluorine or chlorine, especially chlorine. Any alkyl, alkoxy or alkylthio radical of

1 to 4 carbon atoms is preferably of 1 to 2 carbon atoms. Hydroxyalkyl has preferably 2 or 3 carbon atoms. Preferably the hydroxy group is attached to a carbon atom other than the carbon atom adjacent to the nitrogen atom.

The alkoxy moiety in alkoxyalkyl is preferably located in the terminal position of the alkylene chain which preferably has 2 or 3, especially 2 carbon atoms. The alkoxy radical in alkoxyalkyl is preferably methoxy. The alkanoyl group has preferably 2 to 4 carbon atoms, especially 2 carbon atoms.

$R_1$ is preferably hydrogen or halogen in the ring positions marked a and b.

$R_2$ is preferably alkyl.

$R_3$ is preferably alkyl, halogen or alkylthio.

$R_4$ is preferably hydrogen.

$R_5$ is preferably halogen.

The present invention also provides a process for the production of a compound of formula I as defined above, which comprises reacting a compound of formula II,

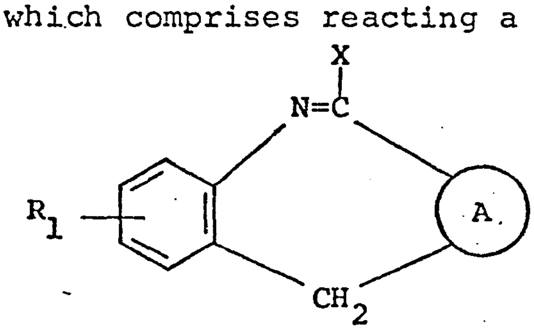

II

wherein $R_1$ and A are as defined above and

X is a leaving group,

with a compound of formula III,

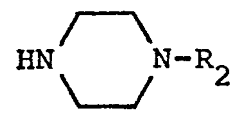

III

wherein $R_2$ is as defined above.

The reaction may be effected in conventional manner for the production of similar thienobenzazepines.

X is preferably chlorine.

The process may be conveniently effected at a temperature of from 50° to 170° C in an inert organic solvent such as xylene or dioxane.

The starting material of formula II may be prepared in known manner, e.g. as described herein, for example via the corresponding lactam, e.g. by reaction with phosphoroxychloride.

Free base forms of the compounds of formula I may be converted into acid addition salt forms in conventional manner and vice versa. Suitable acids are hydrochloric acid, hydrobromic acid, oxalic acid, maleic acid and methanesulphonic acid.

Insofar as the production of starting materials is not particularly described these compounds are known or may be produced in analogous manner to known compounds.

In the following Examples the temperatures given are in degrees Centigrade and are uncorrected.

Example 1: 2-Methyl-4-(4-methyl-1-piperazinyl)-10H-thieno[3,2-c][1]benzazepine [A = AI]

10 g of 2-methyl-4,5-dihydro-10H-thieno[3,2-c][1]benzazepin-4-one, 70 ml of phosphoroxychloride and 3 ml of N,N-dimethylaniline are boiled for 3 hours. The resulting solution of the imidochloride of the lactam is evaporated to dryness and the residue evaporated twice more after the addition of xylene. The residue is then boiled for 4 hours with 10 ml of dioxane and 40 ml of N-methylpiperazine. The resulting mixture is evaporated to dryness. The residue is treated with ice-water and ammonia and extracted with ether. The ether phase is washed twice with water and treated with 1N acetic acid. The acetic acid phase is made alkaline with concentrated ammonia and extracted with ether. The ether phase is washed with water dried over anhydrous sodium sulphate and evaporated. The residue is crystallized from ether/petrolether to give the title compound, m.p. 138-140°.

The starting material 2-methyl-4,5-dihydro-10H-thieno[3,2-c][1]benzazepin-4-one may be obtained as follows:

20 g of 2-(2-isocyanato-benzyl)-5-methylthiophene and 200 g polyphosphoric acid are heated for 1 hour

at 110°. The reaction mixture is then under cooling made alkaline with concentrated ammonia. The resulting solid is filtered, washed with water and treated with charcoal/acetone. M.p. 218-221°.

In analogous manner to that described in Example 1, the following compounds of formula I may be obtained, wherein A is AI:-

| Example | $R_1$ | $R_2$ | $R_3$ | $R_4$ | m. p. |
|---|---|---|---|---|---|
| 2 | 7-F | $CH_3$ | $CH_3$ | H | 160-162 |
| 3 | 7-Cl | $CH_3$ | $CH_3$ | H | 164-166 |
| 4 | 7-Cl | $CH_3$ | H | $CH_3$ | 204-206 |
| 5 | 7-F | $CH_3$ | H | $CH_3$ | 102-105 |
| 6 | 7-F | $CH_3$ | Cl | H | 142-145 |
| 7 | 7-Cl | $CH_3$ | Cl | H | 182-185 |
| 8 | H | H | $CH_3$ | H | 167-170 |
| 9 | H | H | Cl | H | 148-150 |
| 10 | H | $CH_3$ | Cl | H | 137-139 |
| 11 | H | H | H | $CH_3$ | 172-176 |
| 12 | H | $CH_3$ | H | $CH_3$ | 146-148 |
| 13 | H | $CH_2CH_2OH$ | $CH_3$ | H | 119-122 |
| 14 | H | $CH_3$ | $C_2H_5$ | H | 121-124 |
| 15 | H | $CH_3$ | $SCH_3$ | H | 107-110 |
| 16 | 7-Cl | $CH_3$ | $SCH_3$ | H | |
| 17 | H | $CH_3$ | $SO_2CH_3$ | H | 214-217 |
| 18 | H | $CH_3$ | $i-C_3H_7$ | H | |
| 19 | H | $CH_3$ | Br | H | |
| 20 | 7-Cl | $CH_3$ | $i-C_3H_7$ | H | |

In analogous manner to that described in Example 1, the following compounds of formula I may be obtained wherein A is AII:

| Example | $R_1$ | $R_2$ | $R_5$ | m. p. |
|---|---|---|---|---|
| 21 | H | $CH_3$ | $CH_3$ | 109-112 |
| 22 | 7-Cl | $CH_3$ | $CH_3$ | 158-160 |
| 23 | 7-F | $CH_3$ | $CH_3$ | 144-146 |
| 24 | H | H | $CH_3$ | 124-129 |
| 25 | H | $CH_3$ | Cl | 121-123 |
| 26 | 7-Cl | $CH_3$ | Cl | |
| 27 | 7-F | $CH_3$ | Cl | |

The compounds of formula I exhibit pharmacological activity. In particular, they exhibit sedative, antipsychotic and neuroleptic activity, as indicated in standard tests. For example, in one standard test an inhibition of spontaneous motility is observed in mice on p.o. administration of from 0.1 to 5 mg/kg animal body weight of the compounds in accordance with the principles of Caviezel and Baillod [Pharm.Acta Helv. (1958), 33, 465-484]. Additionally, the compounds on administration of from 0.01 to 30 mg/kg i.p. to mice inhibit the hyper-motility induced by 4,α-dimethyl-m-tyramine in a test carried out according to the principles of J.B. Lassen, Psychopharmacologia 37, 331-340 (1974).

The compounds are therefore indicated for use as

sedative, antipsychotic and/or neuroleptic agents. As sedatives and/or neuroleptics an indicated daily dose is from about 5 to about 100 mg, conveniently administered in divided doses 2 to 4 times a day in unit dosage form containing from about 1 to about 50 mg, or in sustained release form.

As antipsychotics an indicated daily dose is from about 5 to about 500 mg and dosage forms may contain from about 1 to about 250 mg.

Additionally, the compounds exhibit muscle relaxing activity as indicated in standard tests, e.g. in rabbits on i.v. administration of from 0.001 to 0.1 mg/kg animal body weight a significant muscle relaxing effect is observed in accordance with the method of Teschendorf et al., Arch.Exp.Pharmacol. 266, 467-468 (1970) and an inhibition of the rigor induced by Thalamonal is observed in rats on i.v. administration of from about 0.001 to about 5 mg/kg.

The compounds are therefore further indicated for use as muscle relaxants. For this use an indicated daily dose is from about 5 mg to about 100 mg, conveniently given in divided doses 2 to 4 times a day in unit dosage

form containing from about 1.25 to about 50 mg or in sustained release form.

The compounds of formula I, especially those wherein $R_2$ is hydrogen, exhibit anti-depressant activity as indicated in standard tests, e.g. an inhibition of tetrabenazine-induced catalepsy and ptosis in rats on intraperitoneal administration of from 5 to 15 mg/kg animal body weight in accordance with the method described by Stille (Arzneimittel-Forsch. 1964, 14, 534).

The compounds are therefore indicated for use as anti-depressant agents. For this use an indicated daily dose is from about 5 to about 500 mg, conveniently administered in divided doses 2 to 4 times a day, in unit dosage form containing from about 2 to about 250 mg or in sustained release form.

The compounds of formula I may be administered in pharmaceutically acceptable acid addition salt form. Such acid addition salt forms exhibit the same order of activity as the free base forms. The present invention also provides a pharmaceutical composition comprising a compound of formula I, in free base form or in pharmaceutically acceptable acid addition salt form,

in association with a pharmaceutical carrier or diluent. Such compositions may be in the form of, for example, a solution or a tablet.

The preferred compounds are those of Examples 1, 3, 10, 21 and 22. The preferred utility is the neuroleptic utility.

In one group of compounds A is a radical of formula AI, $R_1$ is hydrogen, halogen, alkyl, alkoxy or alkylthio, each of 1 to 4 carbon atoms, $R_2$ is hydrogen, alkyl of 1 to 4 carbon atoms, hydroxyalkyl with a maximum of 4 carbon atoms, which may be acetylated or alkoxyalkyl with a maximum of 6 carbon atoms, $R_3$ is hydrogen or alkyl of 1 to 3 carbon atoms or halogen and $R_4$ is hydrogen or alkyl of 1 to 3 carbon atoms, subject to the proviso above.

Conveniently $R_1$ is hydrogen or chlorine or fluorine in the 7-position, $R_2$ is methyl, $R_3$ is hydrogen or methyl or chlorine, $R_4$ is hydrogen or methyl, subject to the proviso above.

In another group of compounds A is a radical of formula AII. $R_1$ is hydrogen, halogen, trifluoromethyl, alkyl, alkoxy or alkylthio, each of 1 to 4 carbon atoms, in position a or b, $R_2$ is hydrogen, alkyl of 1 to 4 carbon atoms, hydroxyalkyl with a maximum of 4 carbon atoms, which may be acetylated or alkoxyalkyl with a maximum of 6 carbon atoms and $R_5$ is halogen or alkyl of 1 to 3 carbon atoms.

WHAT WE CLAIM IS:

1. A compound of formula I,

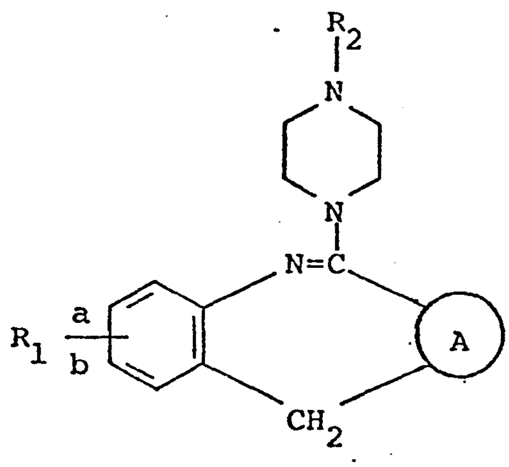

I

wherein $R_1$ is hydrogen, halogen, trifluoromethyl or alkyl, alkoxy, alkylthio, alkylsulfinyl or alkylsulfonyl, each of 1 to 4 carbon atoms,

$R_2$ is hydrogen, alkyl of 1 to 4 carbon atoms, hydroxyalkyl with a maximum of 4 carbon atoms, which may be acylated by an alkanoyl group of 2 to 18 carbon atoms or alkoxyalkyl with a maximum of 6 carbon atoms, and

A is a radical of formula AI or AII

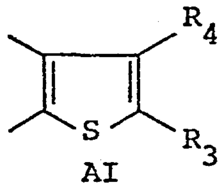

AI

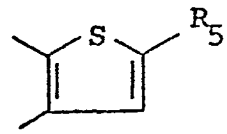

AII

wherein $R_3$ is hydrogen, halogen, trifluoromethylthio, trifluoromethylsulfinyl, trifluoromethylsulfonyl or alkyl, alkoxy, alkylthio, alkylsulfinyl or alkylsulfonyl, each of 1 to 4 carbon atoms,

$R_4$ is hydrogen or alkyl of 1 to 4 carbon atoms, with the proviso that one of $R_3$ and $R_4$ is hydrogen and the other of $R_3$ and $R_4$ is other than hydrogen, and

$R_5$ is halogen or alkyl of 1 to 4 carbon atoms

or an acid addition salt thereof.

2. A process for the production of a compound of formula I, as defined in claim 1, which comprises reacting a compound of formula II,

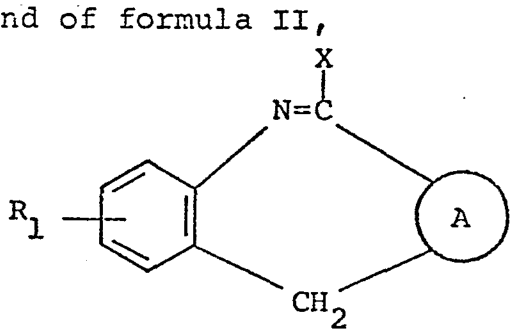

II

wherein $R_1$ and A are as defined in claim 1 and

X is a leaving group,

with a compound of formula III,

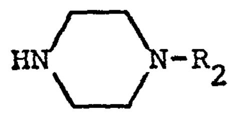

III

wherein $R_2$ is as defined in claim 1.

3. A compound of claim 1, wherein A is a radical of formula AI, $R_1$ is hydrogen, halogen, alkyl, alkoxy or alkylthio, each of 1 to 4 carbon atoms, $R_2$ is hydrogen, alkyl of 1 to 4 carbon atoms, hydroxyalkyl with a maximum of 4 carbon atoms, which may be acetylated or alkoxyalkyl with a maximum of 6 carbon atoms, $R_3$ is hydrogen or alkyl of 1 to 3 carbon atoms or halogen and $R_4$ is hydrogen or alkyl of 1 to 3 carbon atoms with the proviso of claim 1.

4. A compound of claim 3, wherein $R_1$ is hydrogen or chlorine or fluorine in the 7-position, $R_2$ is methyl, $R_3$ is hydrogen or methyl or chlorine, $R_4$ is hydrogen or methyl with the proviso of claim 1.

5. A compound of claim 1, wherein A is a radical of formula AII, $R_1$ is hydrogen, halogen, trifluoromethyl, alkyl, alkoxy or alkylthio, each of 1 to 4 carbon atoms, in position a or b, $R_2$ is hydrogen, alkyl of 1 to 4 carbon atoms, hydroxyalkyl with a maximum of 4 carbon atoms, which may be acetylated or alkoxyalkyl with a maximum of 6 carbon atoms and $R_5$ is halogen or alkyl of 1 to 3 carbon atoms.

6. A compound of claim 1, which is 2-methyl-4-(4-methyl-1-piperazinyl)-10H-thieno[3,2-c][1]benzazepine.

7. A pharmaceutical composition comprising a compound of claim 1 in free base form or in pharmaceutically acceptable acid addition salt form in association with a pharmaceutical carrier or diluent.

3700/IG./GD SANDOZ LTD.

European Patent Office

# EUROPEAN SEARCH REPORT

0001401

Application number

EP 78 10 08

## DOCUMENTS CONSIDERED TO BE RELEVANT

| Category | Citation of document with indication, where appropriate, of relevant passages | Relevant to claim |
|---|---|---|
| A | CH - A - 560 220 (WANDER)<br>* Claims; examples 1-10; column 2, lines 43-54 *<br>-- | 1,5,7 |
| A | CH - A - 585 222 (WANDER)<br>* Claims; examples; column 3, lines 41-50 *<br>-- | 5,7 |
| A | DE - A - 2 511 556 (SANDOZ)<br>* Claims; page 2, second paragraph; page 9, second and third paragraph; examples 1-12 *<br>-- | 1-5,7 |
| A | DE - A - 2 316 438 (WANDER)<br>* Claims; page 2, at the bottom - page 3; page 6 at the bottom; pages 14-15; examples *<br>-- | 1-5,7 |
| A | DE - A - 2 257 443 (WANDER)<br>* Claims; examples; page 2, first paragraph; page 9, last two paragraphs *<br>---- | 1-5,7 |

CLASSIFICATION OF THE APPLICATION (Int. Cl.²)

C 07 D 495/04
A 61 K 31/55//
(C 07 D 495/04;
333/00;223/00)

TECHNICAL FIELDS SEARCHED (Int.Cl.²)

C 07 D 495/04

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 04-12-1978 | NUYTS |

EPO Form 1503.1 06.78